# EUROPEAN PATENT APPLICATION

(11) **EP 2 754 442 A1**
(43) Date of publication of application: **16.07.2014**
(21) Application number: 13150702.2
(22) Date of filing: 09.01.2013
(51) Int. Cl.: A61K 31/5575, A61P 15/04

(54) **Misoprostol for the induction of labour**

(71) Applicant: Ferring B.V., 2132 JX Hoofddorp (NL)
(72) Inventor: The designation of the inventor has not yet been filed
(74) Representative: Gibbs, Richard

(57) **Abstract**

The present invention relates to the use of misoprostol for the induction of labour in a pregnant female, and in particular to the use of a sustained delivery device or insert containing substantially 200 µg misoprostol for intravaginal use. The use encompasses methods of therapy as well as compositions for use in such methods.

## Description

### FIELD OF THE INVENTION

The present invention relates to the use of misoprostol for the induction of labour in a pregnant female, and in particular to the use of a sustained delivery device or insert containing substantially 200 µg misoprostol for intravaginal use. The use encompasses methods of therapy as well as compositions for use in such methods.

### BACKGROUND OF THE INVENTION

The present invention relates to the use of misoprostol for the induction of labour in a pregnant female, and in particular the use of a sustained delivery device or insert containing substantially 200 µg misoprostol for intravaginal use.

Misoprostol is a synthetic analogue of prostaglandin E₁, and has been increasingly used for cervical ripening and labour induction administered both vaginally and orally. In some countries, it is available as a 100 µg or 200 µg tablet, which is quartered or halved and then placed in the vagina every four to six hours. However, splitting tablets does not provide adequate control of dosing of misoprostol, nor is drug release from the tablet fragments steady or well defined.

Our patent application WO2004/029125 discloses a controlled release vaginal pessary comprising misoprostol in a cross-linked polyurethane polymer. Sustained release data in vitro is provided. Our patent application WO2006/013335 discloses that the long term storage properties of such misoprostol cross-linked polyurethane sustained release devices may be improved by maintaining the water content at a low level.

A prostaglandin-containing vaginal pessary has been available for a number of years under the trade mark Propess/Cervidil. It contains 10mg of the PGE2 prostaglandin dinoprostone in a cross-linked polyurethane matrix for sustained vaginal release. The pessary is contained within a net bag and has a retrieval cord or tape, allowing the pessary to be withdrawn once the desired dose has been administered or when the woman reaches an appropriate stage during labour.

Cross-linked polyurethane formulations containing a prostaglandin are also disclosed in US4931288.

Patents US6642278, US2004/044080 and WO2003/011301 disclose other background information.

The normal gestation period in a human female is around 40 weeks. Induction of labour may be considered if the pregnancy progresses beyond the 40 week term without the baby being born. Generally, induction is considered if the pregnancy goes beyond the 41 st or 42nd week. Induction may also be considered for a variety of other medical reasons. The so called "Bishop Score" and "Modified Bishop Score" are pre-labour scoring systems used to assess the progression of labour and/or to determine whether induction of labour will be required. The duration of labour is inversely correlated with the Modified Bishop Score; a score that exceeds 8 describes a patient most likely to achieve a successful vaginal birth. Modified Bishop Scores of less than 4 usually require that a cervical ripening method be used before other methods. The determination of a Bishop Score and/or Modified Bishop Score involves assessing certain factors including cervical dilation, length of cervix, cervical effacement, cervical consistency, cervical position, and foetal station.

Induced labour tends to be more painful for the women and can lead to increased use of analgesics. It is also possible that induction may lead to an increased likelihood of caesarean section delivery for the baby. Medical reasons for the inducement of labour include hypertension or pre-eclampsia in the mother. However, induction may have adverse events, such as uterine tachysystole, foetal heart rate (FHR) irregularities, meconium in amniotic fluid, poor neonatal condition (Apgar score), postpartum haemorrhage, chorioamnionitis, diabetes and poor neonatal respiration.

Misoprostol controlled release pessaries have been investigated for possible clinical use and results are disclosed in a number of references including Powers et al. Journal of Clinical Pharmacology 2008, 48: 26-34, Ewert et al., Obstet Gynecol 2006; 108: 1130-7, Wing et al., J Reprod Med 2008; 53: 695-696, Castaneda et al. American Jn of Obstet Gyneco 2005; 193; 1071-5, Rayburn et al., J Soc Gynecol Investig 2006; 13: 112-7, Pevzner et al, Obstet Gynecol 2009; 114: 261-7, Wing Obster Gynecol 2008; 112: 801-12, Wing et al., Obstet Gynecol 2011; 117: 533-41, Pevzner et al., Obstet Gynecol 2009; 114, 1315-21 and Pevzner et al., European J Obstet Gynecol and Repr Biology 2011: 156, 144-148. Results of clinical trials are also disclosed in our publication WO2011/156812, where the principal basis of comparison is absence of drug or escalating misoprostol dosage. Generally speaking, these studies show improved speed to vaginal delivery using misoprostol 200 µg pessaries without increased rate of caesarean delivery.

The present application is based on the discovery of further surprising benefits of misoprostol - containing controlled release vaginal pessaries.

### SUMMARY OF THE INVENTION

Vaginal inserts containing 200 µg misoprostol may be used to induce labour in female subjects. The present invention is based on the finding that induction of labour by administration of vaginal inserts containing 200 µg misoprostol results in significant benefits (for example reduced labour associated adverse events/improved outcomes) which are not observed when labour is induced by administration of vaginal inserts containing 10mg dinoprostone. These benefits and improved outcomes are described below.

Accordingly, a first aspect of this invention provides a method of reducing
(i) pre-delivery oxytocin use
(ii) the total dose of pre-delivery oxytocin administered;
(iii) the duration of pre-delivery oxytocin use; and
(iv) the maximum dose of oxytocin;
after induction of labour in a female, the method comprising administering intravaginally to the female an insert comprising a cross-linked polyurethane reaction polyurethane product of a polyethylene glycol, a triol and a diisocyanate, the insert containing 200 µg misoprostol;
reductions (i), (ii), (iii) and (iv) being reduced in comparison to the administration of said insert containing 10mg dinoprostone.

Inserts containing 200µg misoprostol or 10 mg dinoprostone may also be referred to as containing a "dose reservoir". For example, inserts containing 200µg misoprostol may be said to comprise a "200µg (dose) reservoir of misoprostol". One of skill will appreciate that the phrase "dose reservoir" may be a reference to the total amount of a therapeutic agent contained within any given delivery device - for example a vaginal insert. Once deployed within a patient, a device may release therapeutic agent from the reservoir. The release may be defined as a controlled release where, for example, predetermined quantities of agent are released from the device over a predetermined period of time or at predetermined intervals. The release may further be defined as a "sustained release" where release of the therapeutic agent in maintained (at a constant or variable rate) throughout the period of deployment.

Oxytocin is a naturally occurring hormone commonly used to induce labour. In the present case, a female to be induced for labour may be administered a single vaginal insert comprising misoprostol for a period of time determined by a clinician. For example, an insert comprising misoprostol may be administered for up to about 24 hours. If after the predetermined time there is no indication that the active phase of labour has begun, oxytocin may be administered. Oxytocin may be administered after completion of a 30-minute waiting period, the waiting period beginning with removal of the vaginal insert.

Oxytocin may be dosed according to, for example, a dose regimen such as, for example a "low-dose" regimen. A starting dose of about 1 mU/min may be used and this may be increased to about 1-4 mU/min every 30 minutes if an active labour pattern has not established. The maximum dose of oxytocin administered may be 30 mU/min.

If labour is induced using vaginal inserts containing 200 µg misoprostol and not 10 mg dinoprostone, significant reductions in the requirement for oxytocin, the duration of oxytocin use, the total oxytocin dose and maximum oxytocin dose are observed.

The effect of a misoprostol containing insert is compared to a dinoprostone-containing insert in the same cross-linked polyurethane. The term "insert" refers to the polyurethane hydrogel sustained delivery device, which may be loaded with drug (misoprostol; or dinoprostone for comparison). The terms MVI or MVI200 refer to a formulated polyurethane insert containing 200 µg misoprostol. The term DVI refers to a formulated polyurethane insert containing 10 mg dinoprostone, which is used as the basis for comparison in the experimental data herein. The drug-containing insert may also be referred to as a pessary. In the experimental data herein the term "insert" is also used to include drug-loaded inserts.

The insert may provide a sustained and/or controlled delivery of misoprostol vaginally to a female patient. Retrieval means may be provided for withdrawal of the insert as a desired time according to clinical need.

A second aspect of this invention provides a method of reducing the time to delivery in a female induced for labour, said method comprising administering intravaginally to the female an insert comprising a cross-linked polyurethane reaction product of a polyethylene glycol, a triol and a diisocyanate, the insert containing 200 µg misoprostol;
the reduced time to delivery, being reduced in comparison to the administration of said insert containing 10 mg dinoprostone.

Delivery of a baby may be vaginally or by caesarean section. Vaginal delivery may be spontaneous or with instrumental assistance. Following induction of labour through administration of an insert containing 200 µg misoprostol, delivery of a baby may occur within about 24 hours or within about 12 hours.

Delivery time may begin with the onset of labour which includes a latent and an active phase. As such, the observed reduction in time to delivery may occur as a consequence of a reduction in the duration of the latent and/or active phase of labour. It should be understood that an insert containing misoprostol may be removed at the onset of active labour.

In addition to reducing the time to delivery, the present inventors have discovered that induction of labour through vaginal administration of an insert containing 200 µg misoprostol (as opposed to 10 mg dinoprostone) greatly increases the likelihood that a female will deliver the baby vaginally. Additionally, or alternatively, following induction of labour by administration of an insert containing 200 µg misoprostol, a vaginal delivery may occur within about 24 hours or within about 12 hours.

Generally, the present invention relates to misoprostol-based inserts (for example MVI 200) for use in inducing labour, wherein induction of labour using misoprostol-based inserts confers benefits and/or a reduction in labour associated adverse events as compared to induction using dinoprostone based inserts (for example DVI).

Induction of labour may be used in a number of clinical situations. By way of example, a female may be induced due to cholestatis, pre-eclampsia, premature rupture of membranes. Additionally, or alternatively, labour may be induced because of foetal macrosomia and/or intrauterine growth restriction. Labour may also be induced because the female is post term (nominally 40 weeks). For example, a post-term female may have been pregnant for anywhere between about 40 and 41 weeks or for a term equal to or greater than 41 weeks.

In females induced for one or more of the abovementioned clinical situations, the inventors have identified a series of benefits associated with induction through administration of an insert containing 200 µg misoprostol, which benefits are not observed when labour is induced using an insert comprising 10 mg dinoprostone.

The inventors have observed that in some of the clinical situations described herein, induction of labour using misoprostol as opposed to dinoprostone based inserts, resulted in reduced incidence of a category II foetal heart rate. A category II foetal heart rate may comprise heart rates which exhibit, for example, evidence of tachycardia, bradycardia, loss of or minimal variability, variable decelerations, and/or prolonged decelerations.

Additionally, the inventors have observed that in some of the clinical situations described herein, induction of labour using misoprostol as opposed to dinoprostone based inserts, results in a reduced likelihood of a newborn being allocated an APGAR score 1 minute after birth of lower than 7. An APGAR (Appearance, Pulse, Grimace, Activity, Respiration) score is used as a means to quickly and reproducibly assess and report the health of a baby following delivery. An APGAR score may be recorded at 1 minute and 5 minutes postpartum. These scores may be referred to as the minute 1 and minute 5 APGAR scores. Generally, a score of 3 or below indicates that the baby is in a critical state whereas a score of between about 4 and about 6 indicates that the baby is only moderately critical. Babies allocated scores of 7 or above are generally regarded as normal.

In some cases, instruments such as forceps or a ventouse are used to deliver a baby. In other cases, it may be necessary to deliver a baby by caesarean section. The inventors have discovered that in some of the clinical situations described herein, induction of labour using misoprostol as opposed to dinoprostone based inserts, resulted in a reduced requirement for instrumented vaginal delivery and/or caesarean delivery.

In some of the clinical situations described herein, induction of labour using misoprostol as opposed to dinoprostone based inserts, resulted in a reduced tocolytic agent/drug use. A tocolytic agent/drug may be used to inhibit, suppress or reduce contractions during labour. Examples of tocolytic agents may include terbutaline or magnesium sulfate.

In some of the clinical situations described herein, induction of labour using misoprostol as opposed to dinoprostone based inserts, resulted in reduced risk of postpartum haemorrhage. Postpartum haemorrhage may be characterised by any significant loss of blood by the female following birth. By way of example, the loss of greater than about 500 ml of blood following a vaginal delivery, or about 1000 ml of blood following caesarean section may be regarded as an occurrence of postpartum haemorrhage. Induction of labour may contribute as a risk factor for postpartum haemorrhage which is the most common cause of perinatal maternal death in the developed world and a major cause of maternal morbidity worldwide. As such, the induction of labour using vaginal inserts containing misoprostol - as opposed to dinoprostone may reduce the risk of the induced female suffering postpartum haemorrhage.

In some of the clinical situations described herein, induction of labour using misoprostol as opposed to dinoprostone based inserts, resulted in a reduced risk of an induced female developing Chorioamnionitis. Chorioamnionitis is caused by a (bacterial) infection and results in inflammation of the amnion and/or chorion (the foetal membranes). Chorioamniontis is known to prolong labour. The signs and/or symptoms of chorioamnionitis may include, for example, a fever (temperature > 37.5°C), uterine tenderness, purulent vaginal discharge and/or persistent maternal or foetal tachycardia.

Any of the clinical situations described herein may cause foetal stress. Intrapartum resuscitation techniques may be used to reverse the hypoxic and acidosis states which may occur when a foetus becomes distressed during labour. In some of the clinical situations described herein, the inventors have observed that induction of labour using misoprostol as opposed to dinoprostone based inserts, resulted in a reduction in the use of intrapartum resuscitation techniques.

Meconium is normally held within the foetus' intestines but occasionally, and often when subjected to stress, the foetus will expel the meconium into the amniotic fluid. If the foetus inhales amniotic fluid contaminated with meconium, respiratory problems may ensue. The inventors have discovered that in certain clinical situations induction of labour using misoprostol as opposed to dinoprostone based inserts, resulted in a reduction in the risk that meconium is expelled by the foetus into the amniotic fluid.

In some of the clinical situations described herein, the inventors have observed that following induction of labour using misoprostol as opposed to dinoprostone based inserts, the risk that a neonate required admission to an intensive care unit (ICU) was significantly reduced.

In all aspects of this invention, the misoprostol-containing insert may be administered by introduction into the female at a point determined by the clinician. The insert may be left *in-situ* until the female enters the active phase of labour. Once the active phase of labour has begun, the misoprostol containing insert may be removed. The misoprostol-containing insert may not be left *in situ* for more than a period of time determined by a clinician.

The female induced for labour may be nulliparous or parous.

The female induced for labour may be hospitalised for the first time.

The misoprostol-containing insert may be administered by introduction into the female at a time determined by the clinician. As such, the dosing period is the time from insertion of the drug-containing insert into the female to removal thereof.

The present invention also relates to uses of the misoprostol-containing insert described herein. For example, the invention provides the misoprostol-containing insert for use in any of the methods described herein as well as misoprostol (for example 200 µg misoprostol) for use in the manufacture of medicaments for use in any of the methods described herein. The present invention may provide an insert comprising a cross-linked polyurethane reaction product of a polyethylene glycol, a triol and a diisocyanate and containing 200 µg misoprostol for use in any of the methods described herein.

### DETAILED DESCRIPTION

Experimental data will now be presented by way of example and with reference to the following figures which show:
- Figure 1:: Kaplan-Meier Plot of Time to Vaginal Delivery (All Parity)
- Figure 2:: Kaplan-Meier Plot of Time to Vaginal Delivery (Nulliparous Subjects)
- Figure 3:: Kaplan-Meier Plot of Time to Vaginal Delivery (Parous Subjects)
- Figure 4:: Kaplan-Meier Plot of Time to Any Delivery (All Parity)
- Figure 5:: Kaplan-Meier Plot of Time to Active Labour (All Parity)

### Experimental

### Overall Study Design

This was a Phase III, double-blind, randomised, multicentre study of approximately 1,350 subjects at or near term gestation requiring cervical ripening and induction of labour.

Treatment consisted of administration of one randomly assigned MVI 200 or DVI. Nulliparous and parous subjects were randomised to their assigned treatments within their parity cohort in a double-blinded manner. The insert was to be kept in place for 24 hours unless events occurred that necessitated earlier removal (e.g., onset of active labour or intrapartum adverse event (AE)). Oxytocin was permitted after removal of the insert and completion of a 30-minute waiting period, if needed, to augment or induce labour. Enrolment was stratified by site and by parity, and randomization proceeded to ensure that approximately 60% nulliparous subjects and 40% parous subjects were enroled.

### Detailed Design

This Phase III study was a double-blind, randomised study comparing MVI 200 with DVI. DVI (Cervidil^{®} [Forest Laboratories], Propess^{®} [Ferring Pharmaceuticals]) is an appropriate comparator for MVI 200 because it is the most commonly used marketed cervical ripening product available in the US and because it is identical in appearance to the MVI, allowing the study to be double-blinded. DVI is labeled in the US for a single administration of a single dose with removal at 12 hours. However, there is an adequate amount of drug in the reservoir to allow continuous dosing via controlled release for up to 24 hours. Because of this, the product is approved in some European countries for administration up to 24 hours. The FDA agreed to allow dosing of up to 24 hours for the DVI during this study in order to maintain the blinded nature of the study.

The study was randomised in order to prevent bias in the administration of different treatment groups and to attempt to have an even distribution of baseline characteristics across the arms of the study.

Eligible subjects were randomised to receive one of the following treatments: MVI 200 or DVI

Subjects were treated with one vaginal insert for up to 24 hours, one time only.

Intravenous oxytocin was permitted, when required, at least 30 minutes following removal of the study drug assuming no contraindications and active labour not present.

The MVI 200 and the DVI (Cervidil) were manufactured and released by Controlled Therapeutics (Scotland) Ltd.

The MVI had three components:
● a hydrogel polymer base measuring approximately 30 x 10 x 0.8 mm
● 200 mcg reservoir of misoprostol released at a controlled rate
● a retrieval tape consisting of inert woven polyester into which the polymer base was placed

The DVI had three components:
● a hydrogel polymer base measuring approximately 30 x 10 x 0.8 mm
● 10 mg reservoir of dinoprostone released at approximately 0.3 mg/hour
● a retrieval tape consisting of inert woven polyester into which the polymer base was placed.
Batch number information is provided in Table 1.

**Table 1: Investigational Drug (MVI and DVI) Batch Numbers**

| **Investigational Drug/Dose** | **Batch No.** | **Expiry Date** |
|---|---|---|
| MVI 200 mcg | MS10006 | 31 July 2013 |
| DVI 10 mg (Cervidil) | MA10K02/1 | 30 June 2013 |

For both the MVI and DVI, the polymer base was designed to absorb fluid from the vagina. As the polymer hydrates and swells, it creates a concentration gradient leading to a sustained release of misoprostol or dinoprostone for up to 24 hours. The polymer was a cross-linked polyurethane.

The MVI and DVI study drug inserts and packaging were identical in appearance (double-blinded). Each study drug kit consisted of a foil sachet with a preprinted subject number detailed on the label. The subject number differentiated study drug intended for nulliparous subjects from that intended for parous subjects. A second self-adhesive label identical to that found on the study drug foil sachet was attached to the study drug foil label. The second self-adhesive label was placed on the study drug accountability form for that subject and kept with the study source documents.

The study drug kits were stored in a freezer. If unopened study drug was not used following removal from the freezer, it could have been returned to the freezer for use at a later date. The study drug could have been removed from and returned to the freezer multiple times as long as it was unopened and the total cumulative time outside the freezer was not more than 24 hours. Any study drug remaining out of the freezer for more than a total of 24 hours was discarded and its destruction documented.

### Selection and Timing of Dose for Each Patient

Subjects were randomised to receive one of the following in a double-blind manner: MVI 200 or DVI.

One randomised study drug was administered to each subject by the Investigator or qualified designee. The insert was placed high in the posterior vaginal fornix and positioned transversely. A minimal quantity of water-soluble lubricant could have been used to aid placement of the study drug. The insert was not prewetted or pre-swelled prior to insertion and obstetric cream was not used.

The subject remained in bed for at least 30 minutes after insertion to ensure that sufficient time was provided for the insert to hydrate and start to swell.

The subject was instructed to use caution when using the toilet or washing to avoid inadvertent removal of the insert.

Subjects were treated with study drug for up to 24 hours. The study drug was removed before 24 hours if there was clinical concern for the wellbeing of mother or baby or if an adverse event (AE) occurred:
If the study drug fell out of the vagina spontaneously or was mistakenly removed early, it was not replaced. At the time of removal, an obstetrician, midwife, obstetric nurse, or other qualified site staff removed the insert by gently pulling on the retrieval tape.

### Oxytocin Use

Oxytocin use was not permitted within 7 days prior to study drug administration and while the study drug was *in situ.*

Intravenous oxytocin was permitted, when required, at least 30 minutes following removal of the study drug, assuming no contraindications and no active Labour. Earlier administration was permitted, if required, for treatment of an emergency situation.

### Onset of Active Labour and Delivery

The date and time of onset of active labour were to be recorded throughout the treatment period. Active labour was defined as progressive cervical dilatation to 4 cm with any frequency of contractions OR rhythmic, firm, adequate, quality uterine contractions causing progressive cervical change occurring at a frequency of three or more in 10 minutes and lasting 45 seconds or more.

At time of delivery of neonate, the following were recorded:
● Mode of delivery (spontaneous vaginal, instrumented vaginal, or caesarean)
● If caesarean delivery, the reason for the caesarean delivery was recorded.
● Date and time of delivery of neonate.

### Adverse Events

An AE was defined as any untoward medical occurrence in a patient or clinical trial subject administered a medicinal product and that does not necessarily have a causal relationship with this treatment.

Subjects were questioned and observed for evidence of AEs, whether or not related to study drug.

Adverse events were collected through hospital discharge following delivery. Adverse events that occurred during the labour and delivery (L&D) period were categorised as intrapartum AEs. Following delivery, AEs were categorised as postpartum (maternal) or neonatal events.

### Summaries of Adverse Event Incidence Rates

Averse events were summarised by system organ class and preferred term for intrapartum, postpartum, and neonate events without regard to relationship to study drug.

### Summary of Outcomes and Adverse Events of Special Interest

Safety assessments were also summarised for Outcomes and AEs of Special Interest. Treatment groups were compared using Fisher's exact tests for each of these outcomes or events. However, there was no correction for multiplicity; therefore, p-values should be interpreted with caution.

### Efficacy Results and Tabulations of Individual Patient Data

### Analysis of Efficacy

### Primary Efficacy Endpoint: Time to Vaginal Delivery During the First Hospitalisation

Time to vaginal delivery during first hospitalisation was statistically significantly shorter for MVI 200 subjects (median 1292.00 minutes [21.5 hours]) compared with DVI subjects (median 1968.50 minutes [32.8 hours]) (p<0.001). Time to vaginal delivery during first hospitalisation was also statistically significantly shorter in MVI 200 subjects compared with DVI subjects among the subsets of nulliparous subjects (p<0.001) and parous subjects (p<0.001). Kaplan Meier estimates of time to vaginal delivery are presented in Table 2.

**Table 2: Kaplan-Meier Estimates of Time to Vaginal Delivery (ITT/Safety Population)**

| **Time From Study Drug Administration to Vaginal Delivery (minutes)** | **MVI 200 (N=678)** | **DVI (N=680)** |
|---|---|---|
| Any parity | | |
| N | 678 | 680 |
| Median | 1292.00 (21.5 hours) | 1968.50 (32.8 hours) |
| 95% CI¹ | (1200.00, 1402.00) (20 hours, 23.4 hours) | (1812.00, 2093.00) (30.2 hours, 34.9 hours) |
| p-value¹ | <0.001 | |
| Number (%) of censored subjects² | 181 (26.7) | 193 (28.4) |
| Caesarean delivery (imputed value: 6548 minutes) | 176 (26.0) | 184 (27.1) |
| Discharged prior to delivery (imputed value: 4571 minutes) | 5 (0.7) | 9 (1.3) |

| Nulliparous subjects | | |
|---|---|---|
| N | 441 | 451 |
| Median | 1750.00 (29.2 hours) | 2586.00 (43.1 hours) |
| 95% CI¹ | (1524.00, 1963.00) (25.4 hours, 32.7 hours) | (2272.00, 2926.00) (37.9 hours, 48.8 hours) |
| p-value¹ | <0.001 | |
| Number (%) of censored subjects² | 156 (35.4) | 176 (39.0) |
| Caesarean delivery (imputed value: 6548 minutes) | 152 (34.5) | 168 (37.3) |
| Discharged prior to delivery (imputed value: 4571 minutes) | 4 (0.9) | 8 (1.8) |

| Parous subjects | | |
|---|---|---|
| N | 237 | 229 |
| Median | 802.00 (13.4 hours) | 1203.00 (20.1 hours) |
| 95% CI¹ | (748.00, 885.00) (12.5 hours, 14.75 hours) | (1065.00, 1368.00) (17.75 hours, 22.8 hours) |
| p-value¹ | <0.001 | |
| Number (%) of censored subjects² | 25 (10.5) | 17 (7.4) |
| Caesarean delivery (imputed value: 4346 minutes) | 24 (10.1) | 16 (7.0) |
| Discharged prior to delivery (imputed value: 1642 minutes) | 1 (0.4) | 1 (0.4) |

| | | |
|---|---|---|
| ¹Two-sided p-values and CIs were obtained from a Log-Rank Test ² Subjects who had a caesarean delivery during the first hospitalisation were censored using the longest time interval from study drug administration to caesarean delivery during the first hospitalisation, independent of treatment group. Subjects who, in their first hospitalisation, were discharged prior to delivery or withdrew consent prior to delivery were censored using the longest time interval from study drug administration to L&D discharge, independent of treatment group. | | |

Time to caesarean delivery was significantly shorter for MVI 200 subjects (median 1431.5 minutes [23.9 hours]) compared with DVI subjects (median 2077.5 minutes [34.6 hours]) (p<0.001). Time to caesarean delivery was also significantly shorter in MVI 200 subjects compared with DVI subjects among both nulliparous subjects (p<0.001) and parous subjects (p<0.001). The summary of time to caesarean delivery is presented in Table 3.

**Table 3: Summary of Time to Caesarean Delivery During First Hospitalisation (ITT/Safety Population)**

| **Time from Study Drug Administration to Caesarean Delivery During First Hospitalisation (minutes)** | **MVI 200 (N=678)** | **DVI (N=680)** |
|---|---|---|
| Any parity | | |
| n | 176 | 184 |
| Median | 1431.5 (23.9 hours) | 2077.5 (34.6 hours) |
| Minimum, maximum | 177,6548 (2.95 hours, 109.1 hours) | 182, 5618 (3.1 hours, 93.6 hours) |
| p-value¹ | <0.001 | |

| Nulliparous subjects | | |
|---|---|---|
| n | 152 | 168 |
| Median | 1516.0 (25.3 hours) | 2097.5 (35 hours) |
| Minimum, maximum | 263,6548 (4.4 hours, 109.1 hours) | 182,5618 (3.1 hours, 93.6 hours) |
| p-value¹ | <0.001 | |

| Parous subjects | | |
|---|---|---|
| n | 24 | 16 |
| Median | 1235.5 (20.6 hours) | 1630.0 (27.2 hours) |
| Minimum, maximum | 177,4346 (2.95 hours, 72.4 hours) | 564,3378 (9.4 hours, 56.3 hours) |
| p-value¹ | 0.163 | |

| | | |
|---|---|---|
| ¹ P-value based on a Wilcoxon Rank Sum test. | | |

### Time to Any Delivery (Vaginal or Caesarean) During the First Hospitalisation

Time to any delivery mode (vaginal or caesarean) was significantly shorter in MVI 200 subjects (Kaplan Meier median 1096.50 minutes [18.3 hours]) compared with DVI subjects (Kaplan Meier median 1639.50 minutes [27.3 hours]) (p<0.001). Time to any delivery was also significantly shorter in MVI 200 subjects compared with DVI subjects among both nulliparous subjects (p<0.001) and parous subjects (p<0.001). Kaplan Meier estimates of time to any delivery are presented Table 4.

**Table 4: Kaplan-Meier Estimates of Time to Any Delivery (ITT/Safety Population)**

| **Time From Study Drug Administration to Any Delivery (minutes)** | **MVI 200 (N=678)** | **DVI (N=680)** |
|---|---|---|
| Any parity | | |
| N | 678 | 680 |
| Median | 1096.50 (18.3 hours) | 1639.50 (27.3 hours) |
| 95% CI¹ | (1031.00, 1170.00) (17.1 hours, 19.5 hours) | (1573.00, 1731.00) (26.2 hours, 28.9 hours) |
| p-value¹ | <0.001 | |
| Number (%) of censored subjects² | 5 (0.7) | 9 (1.3) |
| Discharged prior to delivery (imputed value: 4571 minutes) | 5 (0.7) | 9 (1.3) |

| Nulliparous subjects | | |
|---|---|---|
| N | 441 | 451 |
| Median | 1304.00 (21.7 hours) | 1882.00 (31.4 hours) |
| 95% CI¹ | (1203.00, 1376.00) (20.1 hours, 23 hours) | (1764.00, 2016.00) (29.4 hours, 33.6 hours) |
| p-value¹ | <0.001 | |
| Number (%) of censored subjects² | 4 (0.9) | 8 (1.8) |
| Discharged prior to delivery (imputed value: 4571 minutes) | 4 (0.9) | 8 (1.8) |

| Parous subjects | | |
|---|---|---|
| N | 237 | 229 |
| Median | 777.00 (12.95 hours) | 1155.00 (19.25 hours) |
| 95% CI¹ | (732.00, 823.00) (12.2 hours, 13.7 hours) | (1040.00, 1321.00) (17.3 hours, 22 hours) |
| p-value¹ | <0.001 | |
| Number (%) of censored subjects² | 1 (0.4) | 1 (0.4) |
| Discharged prior to delivery (imputed value: 1642 minutes) | 1 (0.4) | 1 (0.4) |

| | | |
|---|---|---|
| ¹ Two-sided p-values and CIs were obtained from a Log-Rank Test. ² Subjects who did not deliver during their first hospitalisation were censored using the longest time interval from study drug administration to L&D discharge, independent of treatment group. | | |

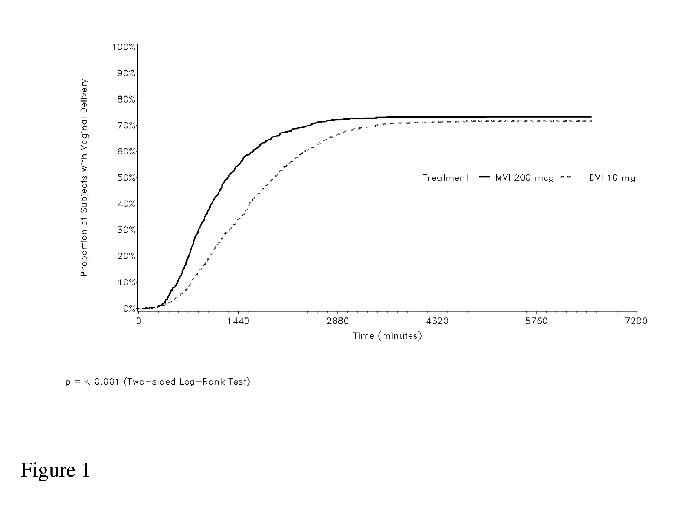

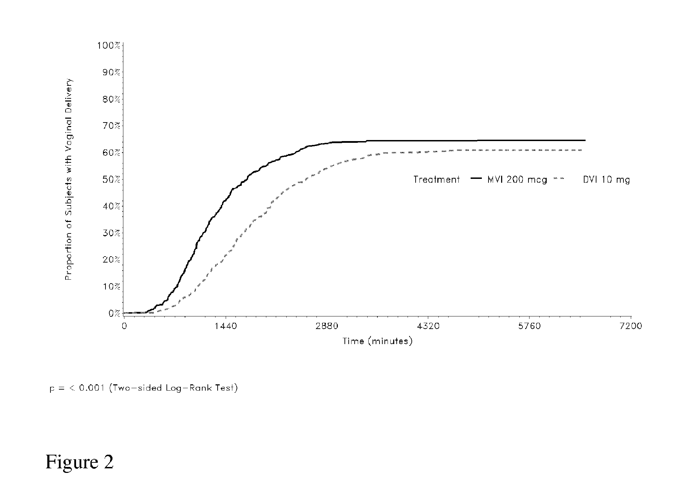

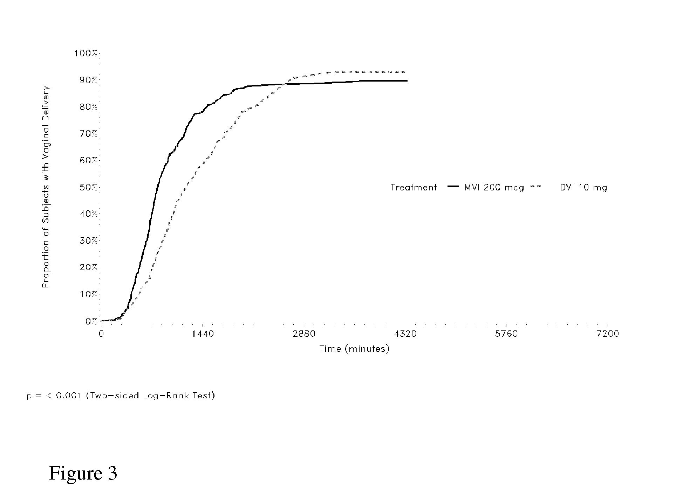

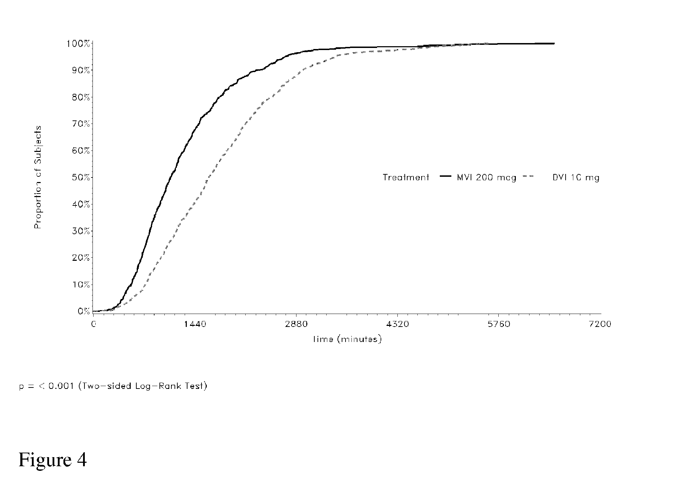

The Kaplan-Meier plot of time to any delivery during the first hospitalisation is presented in Figure 4 (all parity). Kaplan-Meier plots of time to vaginal delivery (all parity), time to vaginal delivery (nulliparous subjects) and time to vaginal delivery (parous subjects) are presented in Figures 1, 2 and 3.

### Time to Active Labour (or duration of latent labour) During the First Hospitalisation

Active labour was defined as progressive cervical dilatation to 4 cm with any frequency of contractions OR rhythmic, firm, adequate, quality uterine contractions causing progressive cervical change occurring at a frequency of three or more in 10 minutes and lasting 45 seconds or more.

Time to active labour was significantly shorter in MVI 200 subjects (median 726.50 minutes [12.1 hours]) compared to DVI subjects (median 1116.50 minutes [18.6 hours]) (p<0.001). Time to active labour was also significantly shorter in MVI 200 subjects compared with DVI subjects among both nulliparous subjects (p<0.001) and parous subjects (p<0.001). Kaplan-Meier estimates of time to active labour are presented in Table 5.

**Table 5: Kaplan-Meier Estimates of Time to Active Labour (ITT/Safety Population)**

| **Time From Study Drug Administration to Active Labour (minutes)** | **MVI 200 (N=678)** | **DVI (N=680)** |
|---|---|---|
| Any parity | | |
| N | 678 | 680 |
| Median | 726.50 (12.1 hours) | 1116.50 (18.6 hours) |
| 95% CI¹ | (719.00, 773.00) (12 hours, 12.9 hours) | (1083.00, 1352.00) (18.1 hours, 22.5 hours) |
| p-value¹ | <0.001 | |
| Number (%) of censored subjects² | 47 (6.9) | 54 (7.9) |
| Discharged without active labour (imputed value: 5618 minutes) | 42 (6.2) | 45 (6.6) |
| Discharged prior to active labour (imputed value: 4571 minutes) | 5 (0.7) | 9 (1.3) |

| Nulliparous subjects | | |
|---|---|---|
| N | 441 | 451 |
| Median | 885.00 (14.8 hours) | 1444.00 (24.1 hours) |
| 95% CI¹ | (810.00, 948.00) (13.5 hours, 15.8 hours) | (1352.00, 1558.00) (22.5 hours, 26 hours) |
| p-value¹ | <0.001 | |
| Number (%) of censored subjects² | 41 (9.3) | 50 (11.1) |
| Discharged without active labour (imputed value: 5618 minutes) | 37 (8.4) | 42 (9.3) |
| Discharged prior to active labour (imputed value: 4571 minutes) | 4 (0.9) | 8 (1.8) |

| Parous subjects | | |
|---|---|---|
| N | 237 | 229 |
| Median | 579.00 (9.7 hours) | 780.00 (13 hours) |
| 95% CI¹ | (535.00, 616.00) (8.92 hours, 10.3 hours) | (715.00, 913.00) (11.9 hours, 15.2 hours) |
| p-value¹ | <0.001 | |
| Number (%) of censored subjects² | 6 (2.5) | 4 (1.7) |
| Discharged without active labour (imputed value: 1451 minutes) | 5 (2.1) | 3 (1.3) |
| Discharged prior to active labour (imputed value: 1642 minutes) | 1 (0.4) | 1 (0.4) |

| | | |
|---|---|---|
| ¹ Two-sided p-values and CIs were obtained from a Log-Rank Test. ² Subjects who did not go into active labour during the first hospitalisation were censored using the longest time interval from study drug administration to delivery during the first hospitalisation, independent of treatment group. Subjects who, in their first hospitalisation, were discharged prior to delivery or withdrew consent prior to delivery were censored using the longest time interval from study drug administration to L&D discharge, independent of treatment group. | | |

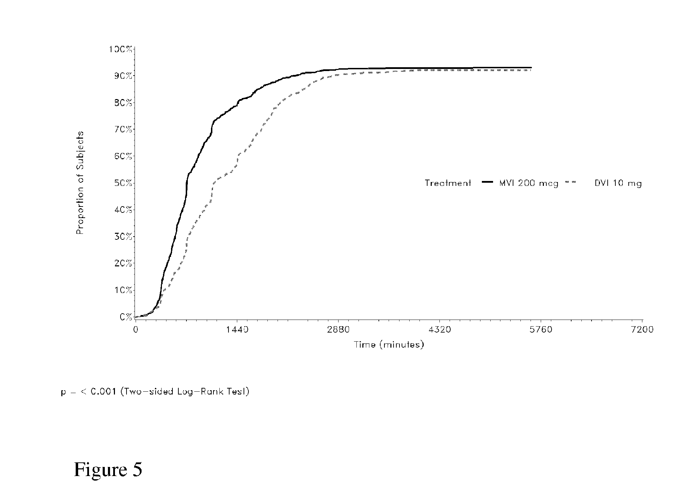

A Kaplan-Meier plot of time to active labour (all parity) is presented in Figure 5

### Incidence of Pre-Delivery Oxytocin Use During the First Hospitalisation

The percentages of subjects requiring pre-delivery oxytocin, pre-delivery oxytocin total dose, duration of pre-delivery oxytocin use, and maximum dose/minute were all lower in the MVI 200 treatment group compared with the DVI treatment group (p<0.001; Table 6).

The statistically significant treatment differences (p≤0.001) were also observed among nulliparous subjects and among parous subjects for these oxytocin parameters.

### Duration of Pre-Delivery Oxytocin Administration for Subjects Who Delivered

### During First Hospitalisation

The percentage of subjects requiring pre-delivery oxytocin was lower for MVI 200 subjects (47.4%/48.1%*) compared with DVI subjects (73.9%/ 74.1%*) (p<0.001) (Table 6). Compared with DVI subjects, MVI 200 subjects had a lower total dose (mean 6.53/4.4* units vs. 8.29 units; p<0.001), shorter duration (mean 498.6/500.6* minutes [8.31/8.34* hours] vs. 657.3/486.62* minutes [10.96/8.11* hours]; p<0.001), and lower maximum dose/minute (mean 10.6 vs. 14.1 mU; p<0.001) of pre-delivery oxytocin (Table 6). Note: * denotes data from a revised analysis of raw data used to obtain the preliminary data (^{a}).

**Table 6: Pre-Delivery Oxytocin Use During First Hospitalisation (ITT/Safety Population)**

| | **MVI 200 (N=678)^{a}** | **MVI 200 (N=678)*** | **DVI (N=680)^{a}** | **DVI (N=680)^{a}** |
|---|---|---|---|---|
| Subjects who delivered during first hospitalisation | 673 | 673 | 671 | 671 |

| Subjects requiring pre-delivery oxytocin¹ | | | | |
|---|---|---|---|---|
| n (%) | 319 (47.4) | 324 (48.1) | 496 (73.9) | 497 (74.1) |
| 95% CI² | (43.57%, 51.25%) | (44.24%, 51.92%) | (70.42%, 77.20%) | 70.58%, 77.35%) |
| p-value³ | <0.001 | <0.001 | | |
| Pre-delivery oxytocin total dose (units) | | 320 | | 496 |
| Mean (SD) | 4.4 (6.53) | 4.4 (6.52) | 7.2(8.29) | 7.2 (8.29) |
| Median | 1.9 | 1.9 | 4.4 | 4.4 |
| Minimum, maximum | 0,48 | 0.48 | 0,50 | 0,50 |
| p-value⁴ | <0.001 | <0.001 | | |
| Duration of pre-delivery oxytocin use (minutes) | | 321 | | |
| Mean (SD) | 498.6 (451.63) | 500.6 (452.08) | 657.3 (487.11) | 657.2 (486.62) |
| Median | 385.0 | 385.0 | 544.5 | 545.0 |
| Minimum, maximum | 1,2625 | 1,2625 | 5,3217 | 5,3217 |
| p-value⁴ | <0.001 | <0.001 | | |
| Maximum dose/minute (mU) | | 321 | | 497 |
| Mean (SD) | 10.6 (8.64) | 10.6 (8.66) | 14.1 (8.97) | 14.1 (8.96) |
| Median | 8.0 | 8.0 | 12.0 | 12.0 |
| Minimum, maximum | 1,42 | 1,42 | 1,42 | 1,42 |
| p-value⁴ | <0.001 | <0.001 | | |

| | | | | |
|---|---|---|---|---|
| ¹ Percentage based on subjects who delivered or received oxytocin during first hospitalisation. ² 95% exact binomial CI. ³ Two-sided p-values were obtained from Fisher's exact tests. ⁴ Two-sided p-values were obtained from one-way ANOVA models. * denotes data from a revised analysis of raw data used to obtain the preliminary data (^{a}). | | | | |

### Incidence of Vaginal Delivery Within 12 Hours of Study Drug Administration

A higher percentage of subjects in the MVI 200 treatment group had vaginal delivery within 12 hours of study drug administration than in the DVI treatment group (19.76% vs. 8.38%, p<0.001; Table 7). The treatment group difference was also statistically significant among nulliparous subjects (p<0.001) and parous subjects (p<0.001).

**Table 7: Incidence of Vaginal Delivery Within 12 Hours of Study Drug Administration (ITT/Safety Population)**

| | **MVI 200 (N=678)^{a}** | **MVI 200 (N=678)*** | **DVI (N=680)^{a}** | **DVI (N=680)^{*}** |
|---|---|---|---|---|
| Any parity | 370 | 678 | 231 | 680 |
| Within 12 hours, n (%) | 134 (19.76) | 134 (19.76) | 57 (8.38) | 57 (8.38) |
| 95% CI¹ | (16.83%, 22.96%) | (16.83%, 22.96%) | (6.41%, 10.72%) | (6.41%, 10.72%) |
| p-value² | <0.001 | <0.001 | | |
| Number of subjects with vaginal delivery | 497 | 499 | 487 | 491 |
| Delivered within 12 hours, n (%)³ | 134 (26.96) | 134 (26.85) | 57 (11.70) | 57 (11.61) |
| Nulliparous | 185 | 441 | 97 | 451 |
| Within 12 hours, n (%) | 40 (9.07) | 40 (9.07) | 12 (2.66) | 12 (2.66) |
| 95% CI¹ | (6.56%, 12.15%) | (6.56%, 12.15%) | (1.38%, 4.60%) | (1.38%, 4.60%) |
| p-value² | <0.001 | <0.001 | | |
| Number of subjects with vaginal delivery | 285 | 286 | 275 | 278 |
| Delivered within 12 hours, n (%)³ | 40 (14.04) | 40 (13.99) | 12 (4.36) | 12 (4.32) |
| Parous | 185 | 237 | 134 | 229 |
| Within 12 hours, n (%) | 94 (39.66) | 94 (39.66) | 45 (19.65) | 45 (19.65) |
| 95% CI¹ | (33.39%, 46.20%) | (33.39%, 46.20%) | (14.71%, 25.40%) | (14.71%, 25.40%) |
| p-value² | <0.001 | <0.001 | | |
| Number of subjects with vaginal delivery | 212 | 213 | 212 | 213 |
| Delivered within 12 hours, n (%)³ | 94 (44.34) | 94 (44.13) | 45 (21.23) | 45 (21.23) |

| | | | | |
|---|---|---|---|---|
| ¹ 95% exact binomial CI. ² Two-sided p-values were obtained from Fisher's exact tests. ³ Percentage based on subjects with vaginal delivery. * denotes data from a revised analysis of raw data used to obtain the preliminary data (^{a}). | | | | |

### Incidence of Any Delivery Within 24 Hours of Study Drug Administration

A higher percentage of subjects in the MVI 200 treatment group had any delivery within 24 hours of study drug administration than in the DVI treatment group (67.70% vs. 40.74%, p<0.001; Table 8). The treatment group difference was also statistically significant among nulliparous subjects (p<0.001) and parous subjects (p<0.001).

**Table 8: Incidence of Any Delivery Within 24 Hours of Study Drug Administration (ITT/Safety Population)**

| | **MVI 200 (N=678)^{a}** | **MVI 200 (N=678)*** | **DVI (N=680)^{a}** | **DVI (N=680)*** |
|---|---|---|---|---|
| Any parity | 459 | 678 | 277 | 680 |
| Within 24 hours, n (%) | 459 (67.70) | 459 (67.70) | 277 (40.74) | 277 (40.74) |
| 95% CI¹ | (64.03%, 71.21%) | (64.03%, 71.21%) | (37.02%, 44.54%) | (37.02%, 44.54%) |
| p-value² | <0.001 | <0.001 | | |
| Nulliparous | 259 | 441 | 137 | 451 |
| Within 24 hours, n (%) | 259 (58.73) | 259 (58.73) | 137 (30.38) | 137 (30.38) |
| 95% CI¹ | (53.98%, 63.37%) | (53.98% ,63.37%) | (26.16%, 34.85%) | (26.16%, 34.85%) |
| p-value² | <0.001 | <0.001 | | |
| Parous | 200 | 237 | 140 | 229 |
| Within 24 hours, n (%) | 200 (84.39) | 200 (84.39) | 140 (61.14) | 140 (61.14) |
| 95% CI¹ | (79.13%, 88.76%) | (79.13%, 88.76%) | (54.49%, 67.49%) | (54.49%, 67.49%) |
| p-value² | <0.001 | <0.001 | | |

| | | | | |
|---|---|---|---|---|
| ¹ 95% exact binomial CI. ² Two-sided p-values were obtained from Fisher's exact tests. * denotes data from a revised analysis of raw data used to obtain the preliminary data (^{a}). | | | | |

### Incidence of Any Delivery Within 12 Hours of Study Drug Administration

A higher percentage of subjects in the MVI 200 treatment group had any delivery within 12 hours of study drug administration than in the DVI treatment group (23.16% vs. 9.26%, p<0.001; Table 9). The treatment group difference was also statistically significant among nulliparous subjects (p<0.001) and parous subjects (p<0.001).

**Table 9: Incidence of Any Delivery Within 12 Hours of Study Drug Administration (ITT/Safety Population)**

| | **MVI 200 (N=678)^{a}** | **MVI 200 (N=678)*** | **DVI (N=680)^{a}** | **DVI (N=680)*** |
|---|---|---|---|---|
| Any parity | 459 | 678 | 277 | 680 |
| Within 12 hours, n (%) | 157 (23.16) | 157 (23.16) | 63 (9.26) | 63 (9.26) |
| 95% CI¹ | (20.03%, 26.52%) | (20.03%, 26.52%) | (7.19%, 11.70%) | (7.19%, 11.70%) |
| p-value² | <0.001 | <0.001 | | |
| Nulliparous | 259 | 441 | 137 | 451 |
| Within 12 hours, n (%) | 57 (12.93) | 57 (12.93) | 16 (3.55) | 16 (3.55) |
| 95% CI¹ | (9.94%, 16.42%) | (9.94%, 16.42%) | 2.04%, 5.70%) | 2.04%, 5.70%) |
| p-value² | <0.001 | <0.001 | | |
| Parous | 200 | 237 | 140 | 229 |
| Within 12 hours, n (%) | 100 (42.19) | 100 (42.19) | 47 (20.52) | 47 (20.52) |
| 95% CI¹ | (35.83%, 48.76%) | (35.83%, 48.76%) | (15.49%, 26.34%) | (15.49%, 26.34%) |
| p-value² | <0.001 | <0.001 | | |

| | | | | |
|---|---|---|---|---|
| ¹ 95% exact binomial CI. ² Two-sided p-values were obtained from Fisher's exact tests. * denotes data from a revised analysis of raw data used to obtain the preliminary data (^{a}). | | | | |

### Incidence of Vaginal Delivery Within 24 Hours of Study Drug Administration

A higher percentage of subjects in the MVI 200 treatment group had vaginal delivery within 24 hours of study drug administration than in the DVI treatment group (54.57% vs. 33.97%, p<0.001; Table 10). The treatment group difference was also statistically significant among nulliparous subjects (p<0.001) and parous subjects (p<0.001).

**Table 10: Incidence of Vaginal Delivery Within 24 Hours of Study Drug Administration (ITT/Safety Population)**

| | **MVI 200 (N=678)^{a}** | **MVI 200 (N=678)*** | **DVI (N=680)^{a}** | **DVI (N=680)*** |
|---|---|---|---|---|
| Any parity | 370 | 678 | 231 | 680 |
| Within 24 hours, n (%) | 370 (54.57) | 370 (54.57) | 231 (33.97) | 231 (33.97) |
| 95% CI¹ | (50.74%, 58.37%) | (50.74%, 58.37%) | (30.41%, 37.67%) | (30.41%, 37.67%) |
| p-value² | <0.001 | <0.001 | | |
| Number of subjects with vaginal delivery | 497 | 499 | 487 | 491 |
| Delivered within 24 hours, n (%)³ | 370 (74.45) | 370 (74.45) | 231 (47.43) | 231 (47.05) |
| Nulliparous | 185 | 441 | 97 | 451 |
| Within 24 hours, n (%) | 185 (41.95) | 185 (41.95) | 97 (21.51) | 97 (21.51) |
| 95% CI¹ | (37.30%, 46.71%) | (37.30%, 46.71%) | (17.80%, 25.59%) | (17.80%, 25.59%) |
| p-value² | <0.001 | <0.001 | | |
| Number of subjects with vaginal delivery | 285 | 286 | 275 | 278 |
| Delivered within 24 hours, n (%)³ | 185 (64.91) | 185 (64.69) | 97 (35.27) | 97 (34.89) |
| Parous | 185 | 237 | 134 | 229 |
| Within 24 hours, n (%) | 94 (39.66) | 185 (78.06) | 45 (19.65) | 134 (58.52) |
| 95% CI¹ | (72.24%, 83.16%) | (72.24%, 83.16%) | (51.84%, 64.97%) | (51.84%, 64.97%) |
| p-value² | <0.001 | <0.001 | | |
| Number of subjects with vaginal delivery | 212 | 213 | 212 | 213 |
| Delivered within 24 hours, n (%)³ | 185 (87.26) | 185 (86.85) | 134 (63.21) | 134 (62.91) |

| | | | | |
|---|---|---|---|---|
| ¹ 95% exact binomial CI. ² Two-sided p-values were obtained from Fisher's exact tests. ³ Percentage based on subjects with vaginal delivery. * denotes data from a revised analysis of raw data used to obtain the preliminary data (^{a}). | | | | |

### Overall Incidence of Vaginal Delivery

No statistically significant difference between treatment groups was observed for the incidence of vaginal delivery during first hospitalisation overall or by parity (Table 11).

**Table 11: Incidence of Vaginal Delivery During First Hospitalisation (ITT/Safety Population)**

| | **MVI 200 (N=678)** | **DVI (N=680)** |
|---|---|---|
| Any parity | | |
| N | 678 | 680 |
| n (%) | 497 (73.30) | 487 (71.62) |
| 95% CI¹ | (69.80%, 76.60%) | (68.07%, 74.98%) |
| p-value² | 0.504 | |

| Nulliparous | | |
|---|---|---|
| N | 441 | 451 |
| n (%) | 285 (64.63) | 275 (60.98) |
| 95% CI¹ | (59.96%, 69.09%) | (56.30%, 65.50%) |
| p-value² | 0.268 | |

| Parous | | |
|---|---|---|
| N | 237 | 229 |
| n (%) | 212 (89.45) | 212 (92.58) |
| 95% CI¹ | (84.82%, 93.06%) | (88.38%, 95.62%) |
| p-value² | 0.261 | |

| | | |
|---|---|---|
| ¹ 95% exact binomial CI. ² Two-sided p-values were obtained from Fisher's exact tests. | | |

Tables **12-21** (below) present data comparing the occurrence of a series of outcomes/adverse events in female subjects administered MVI 200 or DVI. The tables show that induction of labour using MVI 200 confers benefits not observed when labour is induced using DVI.

**Table 12 Reduced category II FHR pattern & reduced risk of intrapartum resuscitation Subjects Induced for Cholestatis**

| | **MVI 200 (N=13)** | **DVI (N=4)** | **Total (N=17)** |
|---|---|---|---|
| Category II FHR Pattern | 2 (15.4%) | 1 (25.0%) | 3 (17.6%) |
| Intrapartum Resuscitation | 1 (7.7%) | 1 (25.0%) | 2 (11.8%) |

**Table 13**

| **Outcomes and Adverse Events of Special Interest** | | | | |
|---|---|---|---|---|
| **Subjects Induced for Preeclampsia** | | | | |
| | | **MVI 200 (N=71)** | **DVI (N=59)** | **Total (N=130)** |
| | Category II FHR Pattern | 10 (14.1%) | 18 (30.5%) | 28 (21.5%) |
| | Intrapartum Resuscitation | 6 (8.5%) | 7 (11.9%) | 13 (10.0%) |
| | Tocolysis Use | 2 (2.8%) | 3 (5.1%) | 5 (3.8%) |
| | Meconium in Amniotic Fluid | 3 (4.2%) | 5 (8.5%) | 8 (6.2%) |
| | Caesarean Delivery During First Hospitalisation | 17 (23.9%) | 17 (28.8%) | 34 (26.2%) |
| | Instrumented Vaginal Delivery During First Hospitalisation | 4 (5.6%) | 7 (11.9%) | 11 (8.5%) |
| | Postpartum Hemorrhage | 4 (5.6%) | 7 (11.9%) | 11 (8.5%) |

**Table 14**

| **Reduced Chorioamnionitis** | | | | |
|---|---|---|---|---|
| **Subjects Induced for Post-Term** | | | | |
| | | **MVI 200 (N=210)** | **DVI (N=227)** | **Total (N=437)** |
| | Chorioamnionitis | 17 (8.1%) | 32(14.1%) | 49(11.2%) |

**Table 15**

| **Reduced neonatal ICU admission** | | | | |
|---|---|---|---|---|
| **Subjects Induced for Post-Term (40 to <41 weeks)** | | | | |
| | | **MVI 200 (N=91)** | **DVI (N=115)** | **Total (N=206)** |
| | Neonatal ICU Admission | 7 (7.7%) | 13 (11.3%) | 20 (9.7%) |

**Table 16**

| **Reduced allocation of low minute 1 APGAR score & reduced neonatal ICU admission** | | | | |
|---|---|---|---|---|
| **Subjects Induced for Post-Term (>= 41 weeks)** | | | | |
| | | **MVI 200 (N=119)** | **DVI (N=112)** | **Total (N=231)** |
| | Minute 1 Apgar Score Low (<7) | 12(10.1%) | 15(13.4%) | 27(11.7%) |
| | Neonatal ICU Admission | 10 (8.4%) | 13(11.6%) | 23(10.0%) |

**Table 17**

| **Reduced instrumented vaginal delivery & reduced neonatal ICU admission** | | | | |
|---|---|---|---|---|
| **Subjects Induced for Intrauterine Growth Restriction** | | | | |
| | | **MVI 200 (N=35)** | **DVI (N=35)** | **Total (N=70)** |
| | Instrumented Vaginal Delivery During First Hospitalisation | 1 (2.9%) | 2 (5.7%) | 3 (4.3%) |
| | Neonatal ICU Admission | 2 (5.7%) | 6 (17.1%) | 8 (11.4%) |

**Table 18**

| **Outcomes and Adverse Events of Special Interest** | | | | |
|---|---|---|---|---|
| **Subjects Induced for Premature Rupture of Membranes** | | | | |
| | | **MVI 200 (N=22)** | **DVI (N=25)** | **Total (N=47)** |
| | Category II FHR Pattern | 5 (22.7%) | 10 (40.0%) | 15 (31.9%) |
| | Intrapartum Resuscitation | 2 (9.1%) | 6 (24.0%) | 8 (17.0%) |
| | Tocolysis Use | 2 (9.1%) | 3 (12.0%) | 5 (10.6%) |
| | Neonatal ICU Admission | 2 (9.1%) | 6 (24.0%) | 8 (17.0%) |

**Table 19**

| **Reduced category II FHR pattern** | | | | |
|---|---|---|---|---|
| **Subjects Induced for Suspected Foetal Macrosomia** | | | | |
| | | **MVI 200 (N=1)** | **DVI (N=3)** | **Total (N=4)** |
| | Category II FHR Pattern | 0 (0.0%) | 3 (100.0%) | 3 (75.0%) |

**Table 20**

| **Reduced allocation of low minute 1 APGAR score & reduced risk of postpartum hemorrhage Subjects with Tocolysis Use** | | | | |
|---|---|---|---|---|
| | | **MVI 200 (N=83)** | **DVI (N=28)** | **Total (N=111)** |
| | Category II FHR Pattern AE | 41 (49.4%) | 20 (71.4%) | 61(55.0%) |
| | Minute 1 Apgar Score Low (<7) | 15(18.1%) | 10(35.7%) | 25(22.5%) |
| | Postpartum Hemorrhage | 7(8.4%) | 4(14.3%) | 11(9.9%) |

**Table 21**

| **Reduced Category II FHR pattern** | | | | |
|---|---|---|---|---|
| **Subjects with Intrapartum Resuscitation** | | | | |
| | | **MVI 200 (N=85)** | **DVI (N=66)** | **Total (N=151)** |
| | Category II FHR Pattern AE | 73(85.9%) | 64(97.0%) | 137(90.7%) |

### Conclusions

● MVI 200 reduced time to vaginal delivery, time to any delivery, and time to onset of active labour compared with DVI.
● MVI 200 reduced pre-delivery oxytocin use compared with DVI.
● MVI 200 had a greater percentage of subjects with vaginal delivery within 12 and 24 hours, any delivery within 12 and 24 hours, and cervical ripening success at 12 hours compared with DVI.
● Results of pharmacoeconomic endpoints demonstrated decreases in duration in L&D, percentage of subjects requiring pre-delivery oxytocin, and duration of maternal hospitalisation with MVI 200 compared with DVI.

## Claims

1. A method of reducing
(i) pre-delivery oxytocin use
(ii) the total dose of pre-delivery oxytocin;
(iii) the duration of pre-delivery oxytocin use; and/or
(iv) the maximum dose of oxytocin
during or after induction of labour in a female, the method comprising administering intravaginally to the female an insert comprising a cross-linked polyurethane reaction product of a polyethylene glycol, a triol and a diisocyanate, the insert containing 200 µg misoprostol;
(i), (ii), (iii) and (iv) being reduced in comparison to the administration of said insert containing 10 mg dinoprostone.

2. A method of reducing the time to delivery in a female induced for labour, said method comprising administering intravaginally to the female an insert comprising a cross-linked polyurethane reaction product of a polyethylene glycol, a triol and a diisocyanate, the insert containing 200 µg misoprostol;
the reduced time to delivery, being reduced in comparison to the administration of said insert containing 10 mg dinoprostone.

3. The method of claim 2, wherein delivery occurs within 24 hours of the administration of the insert containing 200 µg misoprostol or within 12 hours of the administration of the insert containing 200 µg misoprostol.

4. The method of claims 2 or 3 wherein the delivery is a caesarean delivery or a vaginal delivery

5. A method of increasing the likelihood of a vaginal delivery within 24 hours in a female induced for labour, said method comprising administering intravaginally to the female an insert comprising a cross-linked polyurethane reaction product of a polyethylene glycol, a triol and a diisocyanate, the insert containing 200 µg misoprostol;
the increased likelihood of a vaginal delivery within 24 hours being increased in comparison to the likelihood of a vaginal delivery within 24 hours following administration of said insert containing 10 mg dinoprostone.

6. The method of claim 5, wherein the vaginal delivery occurs within 12 hours of the administration of the insert containing 200 µg misoprostol.

7. A method of reducing postpartum neonatal ICU admission after induction of labour in a parous female, said method comprising administering intravaginally to the parous female an insert comprising a cross-linked polyurethane reaction product of a polyethylene glycol, a triol and a diisocyanate, the insert containing 200 µg misoprostol;
the reduction in postpartum neonatal ICU admission, being reduced in comparison to the administration of said insert containing 10 mg dinoprostone.

8. A method of reducing the incidence of category II foetal heart rate pattern and/or intrapartum neonate/foetal resuscitation in a female induced for labour due to cholestatis, said method comprising administering intravaginally to the female an insert comprising a cross-linked polyurethane reaction product of a polyethylene glycol, a triol and a diisocyanate, the insert containing 200 µg misoprostol;
the reduced incidence of category II foetal heart rate pattern and/or intrapartum foetal resuscitation, being reduced in comparison to the administration of said insert containing 10 mg dinoprostone.

9. A method of achieving one or more of:
(i) a reduction in the incidence of category II foetal heart rate pattern;
(ii) a reduction in incidences of intrapartum resuscitation;
(iii) reduced use of tocolytics;
(iv) a reduced incidence of meconium in amniotic fluid;
(v) a reduced requirement for a caesarean delivery during first hospitalisation;
(vi) a reduced requirement for instrumented vaginal delivery during first hospitalisation; and
(vii) a reduced incidence of postpartum haemorrhage.
in a female induced for labour due to pre-eclampsia, said method comprising administering intravaginally to the female an insert comprising a cross-linked polyurethane reaction product of a polyethylene glycol, a triol and a diisocyanate, the insert containing 200 µg misoprostol;
(i), (ii), (iii), (iv), (v) and (vi) being reduced in comparison to the administration of said insert containing 10 mg dinoprostone.

10. A method of reducing postpartum neonatal ICU admission and/or reducing the incidence of chorioamnionitis in a female induced for labour for being post term, said method comprising administering intravaginally to the female an insert comprising a cross-linked polyurethane reaction product of a polyethylene glycol, a triol and a diisocyanate, the insert containing 200 µg misoprostol;
the reduced incidence of postpartum neonatal ICU admission and/or reduced incidence of chorioamnionitis, being reduced in comparison to the administration of said insert containing 10 mg dinoprostone.

11. A method of reducing postpartum neonatal ICU admission in a female induced for labour for being 40 to <41 weeks post term, said method comprising administering intravaginally to the female an insert comprising a cross-linked polyurethane reaction product of a polyethylene glycol, a triol and a diisocyanate, the insert containing 200 µg misoprostol;
the reduced incidence of postpartum neonatal ICU admission, being reduced in comparison to the administration of said insert containing 10 mg dinoprostone.

12. A method of reducing the likelihood of a neonate being allocated a minute 1 agpar score of less than 7 and/or reducing the incidence of postpartum neonatal ICU admission in a female induced for labour for being post term, said method comprising administering intravaginally to the female an insert comprising a cross-linked polyurethane reaction product of a polyethylene glycol, a triol and a diisocyanate, the insert containing 200 µg misoprostol;
the reduced likelihood of a neonate being allocated a minute 1 agpar score of less than 7 and/or reduced incidence of postpartum neonatal ICU admission, being reduced in comparison to the administration of said insert containing 10 mg dinoprostone.

13. The method of claim 12, wherein the female is post term in the range 40 to 41 weeks or in the range greater than or equal to 41 weeks.

14. A method of reducing the requirement for instrumented vaginal delivery and/or reducing postpartum neonatal ICU admission in a female induced for labour for intrauterine growth restriction, said method comprising administering intravaginally to the female an insert comprising a cross-linked polyurethane reaction product of a polyethylene glycol, a triol and a diisocyanate, the insert containing 200 µg misoprostol;
the reduced requirement for instrumented vaginal delivery and/or reduced incidence of postpartum neonatal ICU admission, being reduced in comparison to the administration of said insert containing 10mg dinoprostone.

15. A method of achieving:
(i) reduced incidence of category II foetal heart rate pattern
(ii) reduced intrapartum resuscitation
(iii) reduced use of tocolytics; and
(iv) reduced incidence of postpartum neonatal ICU admission;
in a female induced for labour for premature rupture of membranes, said method comprising administering intravaginally to the female an insert comprising a cross-linked polyurethane reaction product of a polyethylene glycol, a triol and a diisocyanate, the insert containing 200 µg misoprostol;
(i) to (iv) being reduced in comparison to the administration of said insert containing 10mg dinoprostone.

16. A method of reducing the incidence of category II foetal heart rate pattern in a female induced for labour for foetal macrosomia, said method comprising administering intravaginally to the female an insert comprising a cross-linked polyurethane reaction product of a polyethylene glycol, a triol and a diisocyanate, the
the reduced incidence of category II foetal heart rate pattern, being reduced in comparison to the administration of said insert containing 10 mg dinoprostone.

17. A method of reducing one or more of:
(i) a category II FHR pattern;
(ii) postpartum haemorrhage; and
(iii) the allocation of a minute 1 apgar score of <7 to a neonate;
in a female induced for labour and administered a tocolytic agent, said method administering intravaginally to the female an insert comprising a cross-linked polyurethane reaction product of a polyethylene glycol, a triol and a diisocyanate, the insert containing 200 µg misoprostol;
(i), (ii) and/or (iii), being reduced in comparison to the administration of said insert containing 10 mg dinoprostone.

18. A method of reducing a category II FHR pattern in a neonate subjected to intrapartum resuscitation and delivered by female induced for labour, said method administering intravaginally to the female an insert comprising a cross-linked polyurethane reaction product of a polyethylene glycol, a triol and a diisocyanate, the insert containing 200 µg misoprostol;
the reduced category II FHR pattern being reduced in comparison to the administration of said insert containing 10 mg dinoprostone.

19. An insert comprising a cross-linked polyurethane reaction product of a polyethylene glycol, a triol and a diisocyanate and containing 200 µg misoprostol for use in the method of any preceding claim.
